Europäisches Patentamt

European Patent Office

Office européen des brevets

(19)

(11) Publication number: **0 381 168**
**A1**

## (12) EUROPEAN PATENT APPLICATION

(21) Application number: **90101850.7**

(22) Date of filing: **30.01.90**

(51) Int. Cl.5: **C12N 15/53, C12N 9/02**

(30) Priority: **30.01.89 JP 20384/89**
**16.02.89 JP 36928/89**

(43) Date of publication of application:
**08.08.90 Bulletin 90/32**

(84) Designated Contracting States:
**AT BE CH DE DK ES FR GB GR IT LI LU NL SE**

(71) Applicant: **MOCHIDA PHARMACEUTICAL CO., LTD.**
**7, Yotsuya 1-chome**
**Shinjuku-ku Tokyo 160(JP)**

(72) Inventor: **Kamataki, Tetsuya,**
**Minami-8-Jo-Jutaku 401-14**
**1-33, Minami 8 Jo Nishi 23-chome, Chuo-ku**
**Sapporo-shi, Hokkaido(JP)**
Inventor: **Kitada, Mitsukazu, Chuodaiichi 12-23**
**8 Kita 8 Jo Nishi 5-chome**
**Kita-ku, Sapporo-shi, Hokkaido(JP)**
Inventor: **Komori, Masayuki, Kyapitaru**
**Kuraaku 711**
**2 Kita 8 Jo Nishi 6-chome**
**Kita-ku, Sapporo-shi, Hokkaido(JP)**

(74) Representative: **Henkel, Feiler, Hänzel & Partner**
**Möhlstrasse 37**
**D-8000 München 80(DE)**

(54) **Cytochrome P-450 HFLa protein, its DNA sequence and a process for producing said protein.**

(57) DNA sequence of a gene coding for P-450 HFLa protein which is specific to human fetal livers and amino acid sequence deduced from the DNA sequence are provided.

P-450 HFLa protein which contains essentially no other human-originated proteins is produced by means of recombinant DNA technology. Such a high purity renders possible the production of an antibody specific to P-450 HFLa.

EP 0 381 168 A1

**Cytochrome P-450 HFLa Protein, its DNA Sequence and a Process for Producing said Protein**

## BACKGROUND OF THE INVENTION

This invention relates to a gene coding for cytochrome P-450 in human fetal livers (to be referred to as P-450 HFLa hereinafter) which appears specifically in fetal livers and gynecological malignant tumors and P-450 HFLa produced from the said gene.

Cytochrome P-450 is commonly known as a multi-functional enzyme which imparts functions of metabolizing a broad range of biological substances, such as steroids, fatty acids, prostaglandins and vitamin $D_3$, and detoxicating or activating foreign substances, such as drugs and carcinogens, entered into the body.

A broad range of studies have been performed on the cytochrome P-450 using various animal species. Especially, several reports have been published with regard to the correlation between tumor bearing animals and changes in the concentration of the cytochrome P-450, including the following results: 1) when subcutaneous transplantation of Walker carcinosarcoma was applied to a rat, the P-450 content in microsomes in the liver and the activity of drug-metabolizing enzymes decreased as the transplanted cancer cells grew (Jap. J. Pharmac., vol. 18, p. 224, 1968); 2) when a cancer tissue was transplanted into the liver, the P-450 content increased in normal tissues just around the cancer tissue (Kan, Tan, Sui, vol. 15, p. 931, 1987); and 3) the P-450 content in the normal part of the liver was not reduced by a primary liver cancer, but by a metastatic liver cancer (Kan, Tan, Sui, vol. 15, p. 931, 1987). These results, however, do not necessarily show a particular correlation tendency.

The present inventors have directly confirmed the presence of P-450 in human fetuses by partially purifying this enzyme (Biochem. Pharmac., vol. 28, p. 793, 1979). This enzyme was further purified to an electrophoretical homogeneity and named P-450 HFLa (Arch. Biochem. Biophys., vol. 241, p. 275, 1985). In addition, a part of the N-terminal amino acid sequence of the purified P-450 HFLa was identified as follows (Kan. Tan. Sui (the liver, the gall bladder and the pancreas), vol. 15, p. 934, 1987).

Table 1.

| Comparison of N-terminal amino acid sequences of P-450 HFLa and P-450$_{NF}$ | | | | | | | | | |
|---|---|---|---|---|---|---|---|---|---|
| P-450 HFLa | | | | | | | | | |
| 1 | 2 | 3 | 4 | 5 | 6 | 7 | 8 | 9 | 10 |
| X | X | X | ILE | PRO | ASN | LEU | ALA | VAL | GLU |
| P-450$_{NF}$ | | | | | | | | | |
| 1 | 2 | 3 | 4 | 5 | 6 | 7 | 8 | 9 | 10 |
| MET | ALA | LEU | ILE | PRO | ASP | LEU | ALA | MET | GLU |
| Note; "X" in the table indicates the presence of an amino acid which was not able to be identified. | | | | | | | | | |

The N-terminal amino acid sequence of the P-450 HFLa was similar to but different from that of the Guengerich's P-450$_{NF}$.

The P-450 HFLa originated from human fetal livers was chiefly located in fetal livers (Biochem. Biophys. Res. Commun., vol. 131, p. 1154, 1985), showed various activities including 16α-hydroxylation of dehydroepiandrosterone 3-sulfuric ester and was found to be produced in remarkably high frequency in gynecological malignant tumors, especially in ovarian cancer and endometrial cancer (Kan. Tan. Sui, vol. 15, p. 931, 1987).

In consequence, P-450 HFLa has been assumed to be useful as a tumor marker for cancer diagnosis, but, being originated from fetal livers, with a difficulty of obtaining the P-450 HFLa due to a quantitative limitation of fetal livers and also from an ethical point of view. Because of such a difficulty, a large scale production of P-450 HFLa has been expected to be developed by means of recombinant DNA technology.

Elucidation of the amino acid sequence of the P-450 HFLa has also been expected for the purpose of preparing an antibody which is specific to the P-450 HFLa, because a cross reaction has been confirmed between an anti-P-450 HFLa antibodies prepared using a purified preparation of the P-450 HFLa as the antigen obtained from fetal livers and the cytochrome $P-450_{NF}$ which has a similar structure to the P-450 HFLa (Kan. Tan. Sui, vol. 15, p. 931, 1987).


## SUMMARY OF THE INVENTION


An object of the present invention is to provide a cytochrome P-450 HFLa protein essentially free of other proteins of human origin.

Another object of the present invention is to provide said cytochrome P-450 HFLa protein produced by a recombinant host cell.

A still another object of the present invention is to provide a cytochrome P-450 HFLa protein comprising at least a portion of an amino acid sequence represented by sequence [I]:

```
Met Asp Leu Ile Pro Asn Leu Ala Val Glu
Thr Trp Leu Leu Leu Ala Val Ser Leu Ile
Leu Leu Tyr Leu Tyr Gly Thr Arg Thr His
Gly Leu Phe Lys Lys Leu Gly Ile Pro Gly
Pro Thr Pro Leu Pro Phe Leu Gly Asn Ala
Leu Ser Phe Arg Lys Gly Tyr Trp Thr Phe
Asp Met Glu Cys Tyr Lys Lys Tyr Arg Lys
Val Trp Gly Ile Tyr Asp Cys Gln Gln Pro
Met Leu Ala Ile Thr Asp Pro Asp Met Ile
Lys Thr Val Leu Val Lys Glu Cys Tyr Ser
Val Phe Thr Asn Arg Arg Pro Phe Gly Pro
Val Gly Phe Met Lys Asn Ala Ile Ser Ile
Ala Glu Asp Glu Glu Trp Lys Arg Ile Arg
Ser Leu Leu Ser Pro Thr Phe Thr Ser Gly
Lys Leu Lys Glu Met Val Pro Ile Ile Ala
Gln Tyr Gly Asp Val Leu Val Arg Asn Leu
Arg Arg Glu Ala Glu Thr Gly Lys Pro Val
Thr Leu Lys His Val Phe Gly Ala Tyr Ser
Met Asp Val Ile Thr Ser Thr Ser Phe Gly
Val Ser Ile Asp Ser Leu Asn Asn Pro Gln
Asp Pro Phe Val Glu Asn Thr Lys Lys Leu
Leu Arg Phe Asn Pro Leu Asp Pro Phe Val
Leu Ser Ile Lys Val Phe Pro Phe Leu Thr
Pro Ile Leu Glu Ala Leu Asn Ile Thr Val
Phe Pro Arg Lys Val Ile Ser Phe Leu Thr
Lys Ser Val Lys Gln Ile Lys Glu Gly Arg
Leu Lys Glu Thr Gln Lys His Arg Val Asp
Phe Leu Gln Leu Met Ile Asp Ser Gln Asn
Ser Lys Asp Ser Glu Thr His Lys Ala Leu
Ser Asp Leu Glu Leu Met Ala Gln Ser Ile
Ile Phe Ile Phe Ala Gly Tyr Glu Thr Thr
Ser Ser Val Leu Ser Phe Ile Ile Tyr Glu
Leu Ala Thr His Pro Asp Val Gln Gln Lys
Val Gln Lys Glu Ile Asp Thr Val Leu Pro
Asn Lys Ala Pro Pro Thr Tyr Asp Thr Val
Leu Gln Leu Glu Tyr Leu Asp Met Val Val
Asn Glu Thr Leu Arg Leu Phe Pro Val Ala
Met Arg Leu Glu Arg Val Cys Lys Lys Asp
Val Glu Ile Asn Gly Met Phe Ile Pro Lys
Gly Val Val Val Met Ile Pro Ser Tyr Val
Leu His His Asp Pro Lys Tyr Trp Thr Glu
Pro Glu Lys Phe Leu Pro Glu Arg Phe Ser
```

Lys Lys Asn Lys Asp Asn Ile Asp Pro Tyr
Ile Tyr Thr Pro Phe Gly Ser Gly Pro Arg
Asn Cys Ile Gly Met Arg Phe Ala Leu Val
Asn Met Lys Leu Ala Leu Val Arg Val Leu
Gln Asn Phe Ser Phe Lys Pro Cys Lys Glu
Thr Gln Ile Pro Leu Lys Leu Arg Phe Gly
Gly Leu Leu Leu Thr Glu Lys Pro Ile Val
Leu Lys Ala Glu Ser Arg Asp Glu Thr Val
Ser Gly Ala    [ I ]

Yet another object of the present invention is to provide a novel DNA sequence coding for the cytochrome P-450 HFLa protein.

A further object of the present invention is to provide a DNA sequence comprising at least a portion of a DNA sequence represented by sequence [II]:

ATG GAT CTC ATC CCA AAC TTG GCC GTG GAA
ACC TGG CTT CTC CTG GCT GTC AGC CTG ATA
CTC CTC TAT CTA TAT GGA ACC CGT ACA CAT
GGA CTT TTT AAG AAG CTT GGA ATT CCA GGG
CCC ACA CCT CTG CCT TTT TTG GGA AAT GCT
TTG TCC TTC CGT AAG GGC TAT TGG ACG TTT
GAC ATG GAA TGT TAT AAA AAG TAT AGA AAA
GTC TGG GGT ATT TAT GAC TGT CAA CAG CCT
ATG CTG GCT ATC ACA GAT CCC GAC ATG ATC
AAA ACA GTG CTA GTG AAA GAA TGT TAT TCT
GTC TTC ACA AAC CGG AGG CCT TTC GGG CCA
GTG GGA TTT ATG AAA AAT GCC ATC TCT ATA
GCT GAG GAT GAA GAA TGG AAG AGA ATA CGA
TCA TTG CTG TCT CCA ACA TTC ACC AGC GGA
AAA CTC AAG GAG ATG GTC CCT ATC ATT GCC
CAG TAT GGA GAT GTG TTG GTG AGA AAT CTG
AGG CGG GAA GCA GAG ACA GGC AAG CCT GTC
ACC TTG AAA CAC GTC TTT GGG GCC TAC AGC
ATG GAT GTG ATC ACT AGC ACA TCA TTT GGA
GTG AGC ATC GAC TCT CTC AAC AAT CCA CAA
GAC CCC TTT GTG GAA AAC ACC AAG AAG CTT
TTA AGA TTT AAT CCA TTA GAT CCA TTC GTT
CTC TCA ATA AAA GTC TTT CCA TTC CTT ACC
CCA ATT CTT GAA GCA TTA AAT ATC ACT GTG
TTT CCA AGA AAA GTT ATA AGT TTT CTA ACA
AAA TCT GTA AAA CAG ATA AAA GAA GGT CGC
CTC AAA GAG ACA CAA AAG CAC CGA GTG GAT
TTC CTT CAG CTG ATG ATT GAC TCT CAG AAT
TCA AAA GAC TCT GAG ACC CAC AAA GCT CTG
TCT GAT CTG GAG CTC ATG GCC CAA TCA ATT
ATC TTT ATT TTT GCT GGC TAT GAA ACC ACG
AGC AGT GTT CTC TCC TTC ATT ATA TAT GAA
CTG GCC ACT CAC CCT GAT GTC CAG CAG AAA
GTG CAG AAG GAA ATT GAT ACA GTT TTA CCC
AAT AAG GCA CCA CCC ACC TAT GAT ACT GTG
CTA CAG TTG GAG TAT CTT GAC ATG GTG GTG
AAT GAA ACA CTC AGA TTA TTC CCA GTT GCT
ATG AGA CTT GAG AGG GTC TGC AAA AAA GAT
GTT GAA ATC AAT GGG ATG TTT ATT CCC AAA
GGG GTG GTG GTG ATG ATT CCA AGC TAT GTT
CTT CAT CAT GAC CCA AAG TAC TGG ACA GAG
CCT GAG AAG TTC CTC CCT GAA AGG TTC AGT
AAA AAG AAC AAG GAC AAC ATA GAT CCT TAC
ATA TAC ACA CCC TTT GGA AGT GGA CCC AGA
AAC TGC ATT GGC ATG AGG TTT GCT CTC GTG

4

AAC ATG AAA CTT GCT CTA GTC AGA GTC CTT
CAG AAC TTC TCC TTC AAA CCT TGT AAA GAA
ACA CAG ATC CCC CTG AAA TTA CGC TTT GGA
GGA CTT CTT CTA ACA GAA AAA CCC ATT GTT
CTA AAG GCT GAG TCA AGG GAT GAG ACC GTA
AGT GGA GCC TGA     [ II ]

optionally having at least one base substituted by degeneracy of genetic codon.

A still further object of the present invention is to provide a DNA sequence which is complementary to at least a portion of the DNA sequence coding for the cytochrome P-450 HFLa protein or the above-described DNA sequence [II].

Yet further object of the present invention is to provide a process for producing the cytochrome P-450 HFLa protein in which the DNA sequence coding for the cytochrome P-450 HFLa protein is expressed in recombinant host cells.

## BRIEF DESCRIPTION OF THE DRAWINGS

Fig. 1 shows the restriction enzyme map of P-450 HFLa cDNA and the direction of sequencing.

Fig. 2 shows the nucleotide and the deduced amino acid sequences of a gene coding for the P-450 HFLa protein.

## DETAILED DESCRIPTION OF THE INVENTION

The present inventors have isolated a gene coding for the P-450 HFLa protein, determined the nucleotide sequence of said gene and, on the basis of the result, determined amino acid sequence of the P-450 HFLa protein. The present invention has been accomplished as a result of these efforts.

Natural P-450 HFLa can be obtained from the liver of a stillborn child or a fetus taken out due to medical reasons. The excised liver is immediately frozen in liquid nitrogen and cryopreserved at -80° C until just before its use.

Purification of the P-450 HFLa is performed as follows. A portion of the liver of a fetus is homogenized with a 10 mM potassium phosphate buffer (pH 7.25) containing 1.15% KCl, 1mM EDTA, 1mM DTT and 0.4 mM phenylmethylsulfonyl fluoride, and the homogenates are centrifuged at 105,000 x g for 1 hour to obtain P-450 HFLa-containing fraction as the precipitates. The precipitates are washed and centrifuged again. The washed precipitates are solubilized with a potassium phosphate buffer containing sodium cholate and applied onto an ω-aminooctyl-Sepharose 4B column. An active fraction thus obtained is dialyzed against a certain buffer and then subjected to FPLC (Mono S or hydroxylapatite column) to purify the P-450 HFLa in the fraction. Purified P-450 HFLa may be obtained by concentrating, using a PM 30 membrane filter, a fraction which shows a single band by sodium dodecyl sulfate - polyacrylamide gel electrophoresis (SDS-PAGE).

Anti-P-450 HFLa antibodies may be obtained by performing immunization of a P-450 HFLa preparation which has been purified according to the above-described process to an adequate animal, such as a rabbit, using commonly used immunization method.

Extraction of total RNA from fetal livers is performed in accordance with commonly used guanidine thiocyanate method (J. Biol. Chem., vol. 254, p. 9335, 1979). Total RNA is first obtained by the centrifugation of fetal liver homogenates which have been treated with guanidine thiocyanate on a CsCl cushion and then submitted to an affinity column chromatography or a batch process using oligo(dT)-cellulose or poly(U)-sepharose to obtain poly(A)$^+$ RNA (mRNA). Preparation of mRNA may also be performed by other commonly used means, such as a surfactant-treatment in the presence of ribonuclease inhibitor using a vanadium complex or the like or a phenol-treatment. The poly(A)$^+$ RNA thus obtained is fractionated by sucrose density gradient (5 - 25%) centrifugation method using 10 mM Tris-HCl buffer (pH 7.5) containing 10 mM EDTA and 0.2% SDS.

For the purpose of determining a fraction containing the mRNA which is specific to the P-450 HFLa protein, mRNAs in each fraction are translated into proteins *in vitro* and then each of the translated proteins is checked for its physiological activities or the protein specific to the P-450 HFLa is identified using the anti-P-450 HFLa antibodies.

The *in vitro* translation of mRNA may be performed in a cell-free system of rabbit reticulocyte lysate in

the presence of [$^{35}$S] methionine. Translation of mRNA can also be achieved by injecting the mRNA into an oocyte of *Xenopus* sp. or using a wheat germ system. The translated products are mixed with the anti-P-450 HFLa antibodies prepared by the above-described procedure in order to identify the fraction containing P-450 HFLa-specific mRNA by means of an immunoprecipitation method or the like.

Double-stranded cDNA is synthesized by general methods using the mRNA obtained by the above-mentioned procedure as a template, the cDNA thus obtained is ligated into a vector and cDNA library is obtained by transforming *E. coli* or the like with the recombinant vector. For example, synthesis of the double-stranded cDNA from the mRNA may be achieved by a known method using reverse transcriptase or DNA polymerase I (Klenow fragment). The double-stranded cDNA thus synthesized is methylated by *Eco*RI methylase, ligated with an *Eco*RI linker and then cloned into a suitable vector, or the double-stranded cDNA is treated for dC-tailing and then cloned into a dG-tailed vector. The vector for use in this case is preferably a plasmid-type vector such as PBR 322 or a phage-type vector such as λgt 10 and λgt 11.

In the case of the screening of a cDNA library using an expression vector, the screening of the produced proteins may be achieved in accordance with the method of Young and Davis (Proc. Natl. Acad. Sci. USA, vol. 80, p. 1194, 1983) using the anti-P-450 HFLa antibodies. Detection of the immune complex is performed by using a goat anti-rabbit IgG and a peroxidase-conjugated rabbit IgG both on the market. Identification of positive clone may also be performed by colony hybridization or plaque hybridization using an oligonucleotide probe which is synthesized based on the amino acid sequence of the P-450 HFLa protein.

If a cDNA insert of the positive clone is analyzed by agarose gel electrophoresis and the cDNA fragment does not have the entire coding region for the P-450 HFLa protein, a new probe is prepared by radiation-labeling the cDNA fragment by nick translation method and the screening of the cDNA library is started again. As an alternative way in this case, an mRNA fraction which is rich in the mRNA coding for the P-450 HFLa may be determined by northern blotting using the labelled probe and used for the preparation and screening of a new cDNA library.

The P-450 HFLa-coding cDNA thus obtained is subcloned into phage vector M13 mp series in order to obtain single-stranded template, and nucleotide sequence (DNA sequence) of the cDNA is then determined by dideoxy-chain termination method.

The following sequence [II] exemplifies the DNA sequence thus determined.

```
ATG GAT CTC ATC CCA AAC TTG GCC GTG GAA
ACC TGG CTT CTC CTG GCT GTC AGC CTG ATA
CTC CTC TAT CTA TAT GGA ACC CGT ACA CAT
GGA CTT TTT AAG AAG CTT GGA ATT CCA GGG
CCC ACA CCT CTG CCT TTT TTG GGA AAT GCT
TTG TCC TTC CGT AAG GGC TAT TGG ACG TTT
GAC ATG GAA TGT TAT AAA AAG TAT AGA AAA
GTC TGG GGT ATT TAT GAC TGT CAA CAG CCT
ATG CTG GCT ATC ACA GAT CCC GAC ATG ATC
AAA ACA GTG CTA GTG AAA GAA TGT TAT TCT
GTC TTC ACA AAC CGG AGG CCT TTC GGG CCA
GTG GGA TTT ATG AAA AAT GCC ATC TCT ATA
GCT GAG GAT GAA GAA TGG AAG AGA ATA CGA
TCA TTG CTG TCT CCA ACA TTC ACC AGC GGA
AAA CTC AAG GAG ATG GTC CCT ATC ATT GCC
CAG TAT GGA GAT GTG TTG GTG AGA AAT CTG
AGG CGG GAA GCA GAG ACA GGC AAG CCT GTC
ACC TTG AAA CAC GTC TTT GGG GCC TAC AGC
ATG GAT GTG ATC ACT AGC ACA TCA TTT GGA
GTG AGC ATC GAC TCT CTC AAC AAT CCA CAA
GAC CCC TTT GTG GAA AAC ACC AAG AAG CTT
TTA AGA TTT AAT CCA TTA GAT CCA TTC GTT
CTC TCA ATA AAA GTC TTT CCA TTC CTT ACC
CCA ATT CTT GAA GCA TTA AAT ATC ACT GTG
TTT CCA AGA AAA GTT ATA AGT TTT CTA ACA
AAA TCT GTA AAA CAG ATA AAA GAA GGT CGC
CTC AAA GAG ACA CAA AAG CAC CGA GTG GAT
TTC CTT CAG CTG ATG ATT GAC TCT CAG AAT
TCA AAA GAC TCT GAG ACC CAC AAA GCT CTG
```

TCT GAT CTG GAG CTC ATG GCC CAA TCA ATT
ATC TTT ATT TTT GCT GGC TAT GAA ACC ACG
AGC AGT GTT CTC TCC TTC ATT ATA TAT GAA
CTG GCC ACT CAC CCT GAT GTC CAG CAG AAA
GTC CAG AAG GAA ATT GAT ACA GTT TTA CCC
AAT AAG GCA CCA CCC ACC TAT GAT ACT GTG
CTA CAG TTG GAG TAT CTT GAC ATG GTG GTG
AAT GAA ACA CTC AGA TTA TTC CCA GTT GCT
ATG AGA CTT GAG AGG GTC TGC AAA AAA GAT
GTT GAA ATC AAT GGG ATG TTT ATT CCC AAA
GGG GTG GTG GTG ATG ATT CCA AGC TAT GTT
CTT CAT CAT GAC CCA AAG TAC TGG ACA GAG
CCT GAG AAG TTC CTC CCT GAA AGG TTC AGT
AAA AAG AAC AAG GAC AAC ATA GAT CCT TAC
ATA TAC ACA CCC TTT GGA AGT GGA CCC AGA
AAC TGC ATT GGC ATG AGG TTT GCT CTC GTG
AAC ATG AAA CTT GCT CTA GTC AGA GTC CTT
CAG AAC TTC TCC TTC AAA CCT TGT AAA GAA
ACA CAG ATC CCC CTG AAA TTA CGC TTT GGA
GGA CTT CTT CTA ACA GAA AAA CCC ATT GTT
CTA AAG GCT GAG TCA AGG GAT GAG ACC GTA
AGT GGA GCC TGA    [ II ]

The DNA sequence of the present invention may be at least a portion of the DNA sequence shown above as the sequence [II], or a DNA sequence which contains the DNA sequence [II] as a part.

As described above, the DNA sequence of the present invention may be synthesized either by using a template mRNA or by means of organic synthetic chemistry.

In accordance with the degeneracy of genetic codon, at least one base in the DNA sequence of a gene can be replaced with a different base without altering the amino acid sequence of protein produced from the gene. Therefore, the DNA sequence of the present invention may comprise a different nucleotide sequence which is changed by a base substitution originated from the degeneracy of genetic codon. Amino acid sequence deduced from the altered DNA sequence by the base substitution will coincide with the amino acid sequence of the P-450 HFLa protein which is shown later as sequence [I]

According to the present invention, a complementary DNA of the above-mentioned DNA is also provided. According to the present invention, a double-stranded DNA may be formed by complementary linkage of the above-mentioned DNA and its complementary DNA.

Amino acid sequence of the P-450 HFLa protein deduced from the above-cited DNA sequence [II] is shown as the following sequence [I]: Met Asp Leu Ile Pro Asn Leu Ala Val Glu
Thr Trp Leu Leu Leu Ala Val Ser Leu Ile
Leu Leu Tyr Leu Tyr Gly Thr Arg Thr His
Gly Leu Phe Lys Lys Leu Gly Ile Pro Gly
Pro Thr Pro Leu Pro Phe Leu Gly Asn Ala
Leu Ser Phe Arg Lys Gly Tyr Trp Thr Phe
Asp Met Gly Cys Tyr Lys Lys Tyr Arg Lys
Val Trp Glu Ile Tyr Asp Cys Gln Gln Pro
Met Leu Ala Ile Thr Asp Pro Asp Met Ile
Lys Thr Val Leu Val Lys Glu Cys Tyr Ser
Val Phe Thr Asn Arg Arg Pho Phe Gly Pro
Val Gly Phe Met Lys Asn Ala Ile Ser Ile
Ala Glu Asp Glu Glu Trp Lys Arg Ile Arg
Ser Leu Leu Ser Pro Thr Phe Thr Ser Gly
Lys Leu Lys Glu Met Val Pro Ile Ile Ala
Gln Tyr Gly Asp Val Leu Val Arg Asn Leu
Arg Arg Glu Ala Glu Thr Gly Lys Pro Val
Thr Leu Lys His Val Phe Gly Ala Tyr Ser
Met Asp Val Ile Thr Ser Thr Ser Phe Gly
Val Ser Ile Asp Ser Leu Asn Asn Pro Gln
Asp Pro Phe Val Glu Asn Thr Lys Lys Leu
Leu Arg Phe Asn Pro Leu Asp Pro Phe Val

Leu Ser Ile Lys Val Phe Pro Phe Leu Thr
Pro Ile Leu Glu Ala Leu Asn Ile Thr Val
Phe Pro Arg Lys Val Ile Ser Phe Leu Thr
Lys Ser Val Lys Gln Ile Lys Glu Gly Arg
Leu Lys Glu Thr Gln Lys His Arg Val Asp
Phe Leu Gln Leu Met Ile Asp Ser Gln Asn
Ser Lys Asp Ser Glu Thr His Lys Ala Leu
Ser Asp Leu Glu Leu Met Ala Gln Ser Ile
Ile Phe Ile Phe Ala Gly Tyr Glu Thr Thr
Ser Ser Val Leu Ser Phe Ile Ile Tyr Glu
Leu Ala Thr His Pro Asp Val Gln Gln Lys
Val Gln Lys Glu Ile Asp Thr Val Leu Pro
Asn Lys Ala Pro Pro Thr Tyr Asp Thr Val
Leu Gln Leu Glu Tyr Leu Asp Met Val Val
Asn Glu Thr Leu Arg Leu Phe Pro Val Ala
Met Arg Leu Glu Arg Val Cys Lys Lys Asp
Val Glu Ile Asn Gly Met Phe Ile Pro Lys
Gly Val Val Val Met Ile Pro Ser Tyr Val
Leu His His Asp Pro Lys Tyr Trp Thr Glu Pro Glu Lys Phe Leu Pro Glu Arg Phe Ser
Lys Lys Asn Lys Asp Asn Ile Asp Pro Tyr
Ile Tyr Thr Pro Phe Gly Ser Gly Pro Arg
Asn Cys Ile Gly Met Arg Phe Ala Leu Val
Asn Met Lys Leu Ala Leu Val Arg Val Leu
Gln Asn Phe Ser Phe Lys Pro Cys Lys Glu
Thr Gln Ile Pro Leu Lys Leu Arg Phe Gly
Gly Leu Leu Leu Thr Glu Lys Pro Ile Val
Leu Lys Ala Glu Ser Arg Asp Glu Thr Val
Ser Gly Ala    [ I ]

Cytochrome P-450 HFLa protein of the present invention may consist of part or whole amino acid sequence expressed as the sequence [I] or comprise the amino acid sequence [I] as a part of the protein. Also, N-terminal of the cytochrome P-450 HFLa protein of the present invention may contain Met or may not.

It is possible to introduce a spontaneous or an artificial mutation at a site of DNA sequence of a gene and, as a result, a corresponding site of peptide structure derived from the mutated DNA sequence without altering the main activity of the protein produced from the gene. In consequence, it is possible that the cytochrome P-450 HFLa protein of the present invention has a structure of mutated amino acid sequence which is completely homologous to the above-mentioned amino acid sequence.

The cytochrome P-450 HFLa protein containing substantially no other human-originated proteins may be obtained by performing the following order of steps: 1) isolation of a replicable recombinant DNA by means of ligation of the DNA sequence of the present invention into a replicable expression vector; 2) formation of transformants by transforming a microorganism or a cell with the replicable recombinant DNA; 3) selection of the transformant from parent cells of the said microorganism or cell; 4) production of the cytochrome P-450 HFLa protein in the host cells of the selected transformant by culturing the said transformant under certain conditions to induce expression of the said DNA sequence in the host cells; and 5) isolation of the produced protein from the cultured transformant cells.

Such a process for obtaining the cytochrome P-450 HFLa protein from a recombinant host cell is applicable to a large scale manufacture of the said protein and, since said protein contains substantially no other human-originated proteins, an antibody specific to the P-450 HFLa may be prepared using the said protein.

## EXAMPLES

Examples of the present invention are given below by way of illustration, and not by way of limitation.

## Example 1

8

Purification of P-450 HFLa from homogenates of human fetal livers

Purification of P-450 HFLa was performed as follows in accordance with the method of Kitada et al. (Arch. Biochem. Biophys., vol. 241, p. 275, 1985). Composition of each buffer solution used in the purification process is shown below.

Buffer A: Potassium phosphate buffer solution (pH 7.25) containing 1.15% KCl, 1 mM DTT, 1 mM EDTA and 0.4 mM phenylmethylsulfonyl fluoride (PMSF).

Buffer B: Potassium phosphate buffer solution (pH 7.25) containing 20% glycerol, 1 mM DTT, 1 mM EDTA, 0.4 mM PMSF and 0.6% sodium cholate.

Buffer C: Potassium phosphate buffer solution (pH 6.5) containing 20% glycerol, 0.1 mM DTT and 0.2% Emulgen 913 (trade name of a surface active agent produced by Kao Atlas Co).

Buffer D: Potassium phosphate buffer solution (pH 7.4) containing 20% glycerol, 0.1 mM DTT and 0.2% Emulgen 913.

Livers were obtained from stillborn fetuses (20 - 28 weeks) and cryopreserved at -80°C until use. Livers (10 - 15 grams) were homogenized with 100 - 150 ml of 10 mM buffer A. After centrifugation at 105,000 x g for 1 h, the resulting pellet was homogenized with 200 - 300 ml of the 10 mM buffer A and centrifuged at 105,000 x g for 30 min. The resulting pellet was homogenized with 300 - 450 ml of 100 mM buffer B and centrifuged at 105,000 x g for 1 h. The resulting supernatant was applied to a column of $\omega$-aminooctyl-Sepharose 4B which had been equilibrated with 100 mM buffer B. After washing the column with 100 mM buffer B, P-450 HFLa was eluted by the washing of the column with 100 mM potassium phosphate buffer (pH 7.25) containing 20% glycerol, 1 mM DTT, 0.1% sodium cholate and 0.5% Emulgen 913. P-450 HFLa-containing fractions were pooled and dialyzed against 10 mM potassium phosphate buffer (pH 6.5) containing 20% glycerol, 0.2 mM DTT and 0.2% Emulgen 913. Further purification was performed by using an FPLC system equipped with a Mono S column. About 1 mg of protein was applied to Mono S column equilibrated with 10 mM buffer C. After washing of the column with 5 ml of 10 mM buffer C, P-450 HFLa was eluted by the use of a linear gradient of NaCl (0 - 300 mM; total volume, 15 ml) in 10 mM buffer C at a flow rate of 1 ml/min. The elution of P-450 HFLa was monitored at 405 nm. SDS-PAGE was carried out using a portion of each fraction and fractions containing P-450 HFLa were pooled. Ten milliliter portion of the pooled fractions was directly applied to a hydroxylapatite column (9 x 30 mm) equilibrated with 10 mM buffer D. The column was washed with 20 ml of 10 mM buffer D at a flow rate of 1 ml/min and subsequently washed with 5 ml of 50 mM buffer D. P-450 HFLa was then eluted by the use of a linear gradient of potassium phosphate (50 - 300 mM; total volume, 30 ml) at a flow rate of 1 ml/min. Judging from the results of SDS-PAGE and the elution profile monitored at 405 nm, P-450 HFLa-containing fractions were pooled and concentrated using a PM 30 membrane filter.

By the use of this method, P-450 HFLa was purified from homogenates of human fetal livers to a homogeneous level and the purified preparation had an molecular weight of 51,000 as judged by SDS-PAGE.

Example 2

Preparation of anti-P-450 HFLa antibcdies

Anti-P-450 HFLa antibodies were obtained as an antiserum in accordance with the method of Kamataki et al. (Mol. Pharmacol., vol. 12, p. 921, 1976) using the purified P-450 HFLa according to Example 1.

About 0.1 mg of the purified P-450 HFLa was injected together with the complete Freund's adjuvant into a base of two claws of a hind leg and four sinews of an individual of New Zealand rabbit both on the first and eighth days and the rabbit was given a venous injection of the P-450 HFLa which had been dissolved into physiological saline on the 21st day. On the 8th day after the venous injection, blood was collected from an ear to isolate sera according to a general method and the collected sera were used as the antiserum.

## Example 3

### Extraction of total mRNA from human fetal livers

Extraction of total RNA was carried out in accordance with the method of Raymond et al. (J. Biol. Chem., vol. 254, p. 9335, 1979). That is, about 2 g of fetal liver which had been cryopreserved at -80°C was homogenized with 22 ml of 4M guanidine thiocyanate solution (pH 7.0) containing 0.25 mM sodium citrate and 0.1 M β-mercaptoethanol using polytron (trade name of a homogenizer produced by KINEMATICA Co.) and the homogenate was centrifuged at 12,000 x g for 10 min at 20°C. A 5 ml portion of 5.7 M CsCl solution containing 0.1 M EDTA (pH 7.0) was transferred into a sterile centrifugation tube and about 20 ml of the supernatant obtained after the above-mentioned centrifugation was layered over the CsCl solution. After centrifugation of the layered sample at 80,000 x g for 20 h at 20°C, upper layer in the tube was removed carefully using a pipette and the tube was immediately set upside down on a piece of tissue paper and stood still for a while. The pellet thus obtained was suspended in 10 ml of sterile distilled water and mixed well at 55°C for several minutes in order to dissolve the RNA pellet. RNA was precipitated by adding 1/10 volume of 3 M sodium acetate solution (pH 5.2) and 2.5 volume of ethanol to the dissolved RNA solution and leaving overnight at -20°C to obtain total RNA. The yield of the resulting total RNA was 17.0 mg as estimated by A260.

Isolation of mRNA from the total RNA was carried out using oligo(dT)-cellulose column in accordance with the method of Aviv et al. (Proc. Natl. Acad. Sci.USA, vol. 69, p. 1408, 1972). That is, 1 g of oligo(dT)-cellulose resin was suspended in 20 mM Tris-HCl buffer solution (pH 7.5) containing 1 mM EDTA, 0.5M NaCl and 0.1% SDS in order to prepare a column having 4 ml capacity and the column was washed with the same solution. A total of 340 $A_{260}$ units of the total RNA obtained above was dissolved in 50 ml of a TE solution (10 mM Tris-HCl buffer, pH 7.5, and 1 mM EDTA) containing 0.5 M NaCl and 0.1% SDS and applied to the column after a heat denaturation treatment of the solution. The nonabsorbed material was eluted by washing with the same TE solution and poly(A)[+] RNA retained by the column was eluted with 10 ml of a TE solution containing 0.05% SDS. The poly(A)[+] RNA eluted in this way was precipitated by the addition of 1 ml of 3 M sodium acetate (pH 5.2) and 2.5 volume of ethanol. By performing these steps, 1.0 mg of poly(A)[+] RNA (mRNA) was obtained.

The poly(A)[+] RNA thus obtained was fractionated further by sucrose density gradient sedimentation. Sucrose was dissolved in a TE solution containing 10 mM EDTA and 0.2% SDS to make 5 and 25% sucrose solutions. Sucrose density gradient (5 - 25%) was prepared by mixing these two solutions in a centrifugation tube. A total of 500 μg poly(A)[+] RNA was dissolved in 200 μl of sterile distilled water, heated at 65°C for 5 min, cooled rapidly and then layered over the sucrose gradient. After centrifugation of the layered sample at 110,000 x g for 15 h, the mixture in the tube was fractionated at 0.4 ml intervals and mRNA in each fraction was precipitated and recovered by adding ethanol. The size of poly(A)[+] RNA in each fraction was determined using rRNA as the marker.

## Example 4

### Detection of fraction containing P-450 HFLa mRNA

The mRNA obtained according to Example 3 was translated into proteins and the translated proteins were screened using the anti-P-450 HFLa serum obtained according to Example 2. Translation of mRNA (1/20 volume of each fraction) was carried out using a cell-free system of a rabbit reticulocyte lysate in the presence of 25 μ Ci [35S] methionine. The reaction (total volume, 25 μl; 30°C, 90 min) was carried out according to a protocol published from Amersham Co.

After the translation reaction, 1 μl portion of the reaction mixture was used for the measurement of [35S] radioactivity incorporated into the 10% TCA-insoluble fraction and the rest of the reaction mixture was used for the immunoprecipitation reaction with the anti-P-450 HFLa serum. That is, the rest of the reaction mixture was added with 25 μl of 10% SDS, filled up to 250 μl with distilled water, boiled for 5 min and added further with 1 ml of a dilution solution (190 mM NaCl, 50 mM Tris-HCl (pH 7.5), 6 mM EDTA and 2.5% Triton X-100) and 2 $\mu$l of the anti-P-450 HFLa serum. The mixture thus prepared was then incubated overnight at room temperature.

Thereafter, the reaction mixture was centrifuged at 10,000 x g for 5 min and the resulting supernatant was mixed with 10 $\mu$l of protein A-bound Sepharose which had been swollen with distilled water. After incubating the mixture at room temperature for 2 h, the protein A-bound Sepharose resin was precipitated by centrifugation and the pellet was washed with 1 ml of a washing solution containing 150 mM NaCl, 10 mM Tris-HCl buffer (pH 7.5), 5 mM EDTA, 0.1% Triton X-100 and 0.05% SDS. The total washing process was repeated four times. The washed pellet was then added with 25 $\mu$l of an elution solution consisting of 6% SDS, 0.2 M Tris-HCl buffer (pH 7.5), 5 mM EDTA and 2% sucrose. The mixture was boiled for 5 min and the radioactivity in the eluent was measured using a liquid scintillation counter to determine the aimed mRNA fraction.

<div align="center">

### Example 5

</div>

<div align="center">

Construction of $\lambda$ phage cDNA library

</div>

Synthesis of cDNA was carried out in accordance with the method of Gubler and Hoffman (Gene, vol. 25, p. 263, 1983) using the fraction containing P-450 HFLa mRNA which had been determined according to Example 4.

(1) Synthesis of single-stranded cDNA.

A 20 $\mu$l portion of solution containing about 18 $\mu$g of mRNA which had been treated with heat denaturation was mixed with 1 $\mu$l of 100 mM methylmercury hydroxide, stood still for 10 min at room temperature and then mixed further with 2 $\mu$l of 700 mM $\beta$-mercaptoethanol and 1 $\mu$l of ribonuclease inhibitor (40 units). The reaction solution was stood still for another 5 min at room temperature. Synthesis of single-stranded cDNA was carried out by reacting the solution thus obtained at 42°C for 90 min with 50 $\mu$l of a solution containing 200 $\mu$g/ml of oligo(dT)15, 100 mM Tris-HCl buffer (pH 8.3), 40 mM KCl, 10 mM MgCl2, 2 mM dGTP, 2 mM dATP, 2 mM dTTP, 2 mM dCTP (4 $\mu$ Ci [$^{32}$P] dCTP) and 800 units/ml of reverse transcriptase. The reaction was stopped by adding EDTA to 20 mM finally. The products were extracted with a phenol/chloroform solvent system and the macromolecular nucleic acid in the extract was precipitated with 2 M ammonium acetate and ethanol. The amount of single-stranded cDNA thus synthesized was estimated to be 1.47 $\mu$g by assaying incorporated radioactivity into the cDNA.

(2) Synthesis of double-stranded cDNA.

Total amount of the single-stranded cDNA precipitate (1.47 $\mu$g) thus synthesized was dissolved in 20 $\mu$l of sterile distilled water and then filled up to a reaction volume of 100 $\mu$l which consisted of 20 mM Tris-HCl buffer (pH 7.5), 4 mM MgCl2, 10 mM (NH4)2SO4, 100 mM KCl, 0.15 mM $\beta$-NAD, 50 $\mu$g/ml of BSA, 1 mM dATP, 1 mM dGTP, 1 mM dTTP, 1 mM dCTP (10 $\mu$ Ci [$^{32}$P] dCTP), 8.5 units/ml of E. coli RNase H, 230 units/ml of DNA polymerase I and 10 units/ml of E. coli DNA ligase. The mixture was incubated at 12°C for 60 min and then at 25°C for 60 min and the reaction was stopped by adding EDTA to 20 mM finally. The reaction solution was extracted twice with the phenol/chloroform solvent system and then the extract was treated with 2 M ammonium acetate and ethanol in order to precipitate double-stranded cDNA and to remove unreacted substrate. The amount of double-stranded cDNA thus synthesized was estimated to be 1.87 $\mu$g by assaying incorporated radioactivity into the cDNA.

(3) *EcoRI* methylation and ligation of *EcoRI* linker.

The double-stranded cDNA thus obtained (1.87 $\mu$g) was dissolved in 25 $\mu$l of TE solution (pH 8.0) and filled up to a reaction volume of 50 $\mu$l which consisted of 5 mM EDTA, 200 $\mu$g/ml of BSA, 100 $\mu$M of S-adenosyl methionine and 2,000 units/ml of EcoRI methylase. Methylation reaction was carried out by incubating the mixture at 37°C for 30 min and the resulting product was extracted with a phenol/chloroform system and precipitated with ethanol by conventional methods.

The cDNA thus obtained was dissolved in sterile distilled water which was then mixed with 100 mM Tris-HCl buffer (pH 8.0), 10 mM MgCl$_2$, 1 mM dNTPs ("N" corresponds to A, G, T or C), 0.5 mM DTT, 200 $\mu$g/ml of BSA and 300 units/ml of T$_4$ DNA polymelase to their final concentrations. The mixture was incubated at 37°C for 30 min. Ligation with EcoRI linker was carried out by reacting the incubated solution with 1.5 $\mu$g of phosphorylated EcoRI linker at 16°C overnight using a ligation kit (a commercial product of Takara Shuzo). After the reaction, phenol/chloroform extraction and ethanol precipitation were carried out. EcoRI digestion of the precipitated pellet containing the linker-ligated cDNA was carried out at 37°C for 4 h in 50 $\mu$l of a reaction solution containing 50 mM NaCl, 100 mM Tris-HCl buffer (pH 7.5), 7 mM MgCl$_2$, 7 mM $\beta$-mercaptoethanol and 800 units/ml of EcoRI. The EcoRI digest was extracted with phenol/chloroform system, precipitated with ethanol and then applied to a Sepharose CL-4B gel filtration column. Fractions containing the linker-ligated cDNA having 500 bp or more were pooled and precipitated with ethanol. A total of 450 ng of the linker-ligated cDNA was finally recovered.

(4) Introduction of cDNA into vector.

Reaction of 50 ng of the double-stranded cDNA with 1 $\mu$g of $\lambda$gt 11 which had been digested with EcoRI was carried out at 16°C for 6 h in 10 $\mu$l of a TE solution (pH 7.4) containing 10 mM MgCl$_2$, 10 mM DTT, 1 mM ATP, 100 $\mu$g/ml of BSA and 750 units/ml of T$_4$ ligase.

(5) In vitro packaging.

Packaging of the recombinant phage DNA thus obtained was carried out using an in vitro packaging kit (a commercial product of Promega) in accordance with a protocol provided by Promega. That is, 7.5 $\mu$l of the recombinant phage DNA solution was mixed with 37.5 $\mu$l of the packaging extract and the mixture was incubated at 22°C for 2 h. The resulting reaction solution was diluted with 375 $\mu$l of a dilution solution consisting of 0.1M NaCl, 10 mM Tris-HCl buffer (pH 8.0) and 10 mM MgSO$_4$ and stored at 4°C after adding 20 $\mu$l of chloroform.

## Example 6

Screening of cDNA library using anti-P-450 HFLa antibodies

Screening was carried out by modifying the method of Young and Davis (Proc. Natl. Acad. Sci. USA, vol. 80, p. 1194, 1983) as follows.

(1) Formation of phage plaques

E. coli Y 1090 was cultured overnight using a liquid medium which consisted of 10 g/l of tryptone, 5 g/l of yeast extract, 5 g/l of NaCl, 2.5 g/l of MgSO$_4 \cdot$7H$_2$O and 2 g/l of maltose. A 300 $\mu$l portion of the cultured broth was mixed with 10 $\mu$l of the recombinant phage solution which had been prepared according to Example 5 and incubated at 37°C for 20 min in a test tube in order to complete transfection. The incubated solution was mixed with 7.5 ml of a soft agar medium consisting of 10 g/l of tryptone, 5 g/l of NaCl, 2.5 g/l of MgSO$_4 \cdot$7H$_2$O and 7 g/l of agar and the mixture was poured and solidified on a plate medium consisting of 10 g/l of tryptone, 5 g/l of NaCl, 2.5 g/l of MgSO$_4 \cdot$7H$_2$O and 15 g/l of agar wherein the autoclaved medium was supplemented with 100 mg/l of ampicillin. After incubating the plate at 42°C for 3.5 h, a nitrocellulose filter which had been soaked with 10 mM isopropyl-$\beta$-D-thiogaractopyranoside (IPTG) was put on the overlay agar medium and the plate was incubated again at 37°C for 3.5 h in order to obtain phage plaques. As the result, $3 \times 10^5$ plaques were obtained.

(2) Screening using antibodies

The incubated plate was remained at 4°C for 30 min and then the nitrocellulose filter to which expressed hybrid proteins had been adsorbed was removed from its plate. The removed filter was incubated at room temperature for 1 h in 50 ml of a 3% BSA-containing TS buffer consisting of 50 mM Tris-HCl (pH 7.5) and 150 mM NaCl. Two to three filters obtained in this way were transferred in a vinyl bag and treated with 5 ml of TS buffer solution containing 20 μl of anti-P-450 HFLa antiserum as the primary antibody at room temperature for 1 h. These filters were washed three times (5 min for each) with TS buffer solution in order to remove unreacted antibodies. The washed filters were transferred in a fresh vinyl bag and treated in the same manner as in the case of the primary antibody, except for using 10 μl of goat anti-rabbit IgG (0.6 mg/ml) as the secondary antibody and then using 10 μl of peroxidase-conjugated rabbit IgG (1.5 mg/ml) as the tertiary antibody, respectively. These filters were washed in the same manner as in the case of the primary antibody treatment and then soaked for a several minutes in 25 ml of a TS buffer solution containing 10 μl of $H_2O_2$ as a substrate for the enzyme reaction and 6 mg of 3,3′-diaminobenzidine-4-HCl as a color reagent. Two positive clones which reacted to these antisera were obtained based on the coloring reaction.

(3) Purification of plaques

The soft agar which contained plaques of positive clones according to the above antibody-aided screening process was collected using a Pasteur pipette and suspended in 1 ml of SM medium consisting of 5.8 g/l of NaCl, 2 g/l of $MgSO_4 \cdot 7H_2O$, 50 mM of Tris-HCl buffer (pH 7.5), 0.01% gelatine and a small quantity of chloroform. The suspension was remained at 4°C for 30 min in order to disperse phages. The phage suspension was diluted with SM medium and treated in accordance with the process (1) of Example 6 in order to obtain phage plaques. A single positive clone was obtained from the phage plaques after several repetitions of the screening process (2) of Example 6. Finally the positive plaque was suspended in SM medium and used as purified phage.

Example 7

Analysis of positive clone

*E. coli* Y 1088 was cultured overnight using the same liquid medium used in Example 6 for the culturing of Y 1090, 1 ml of the cultured broth was mixed with 50 μl of the purified phage suspension obtained according to the process (3) of Example 6 and the mixture was incubated at 37°C for 20 min in order to complete transfection. To the incubated mixture was added 25 ml of T medium which consisted of 10 g/l of tryptone, 2.5 g/l of NaCl and 2.5 g/l of $MgSO_4 \cdot 7H_2O$ and the resulting mixture was cultured on a shaker at 37°C for 4 to 5 h in order to obtain lysate of the host cells. The lysate was centrifuged at 3,000 rpm for 10 min at 4°C and the resulting supernatant was transferred into another centrifugation tube. To the supernatant in the tube was added 10 ml of DEAE-LB suspension solution (DE 52 : LB medium = 1 : 2) wherein the LB medium consisted of 10 g/l of tryptone, 5 g/l of yeast extract and 5 g/l of NaCl. The supernatant and the suspension solution were mixed by rolling the tube gently and then centrifuged at 3,000 rpm for 10 min at 4°C. Resulting supernatant was transferred into another tube, mixed with 1/10 volume of 3 M sodium acetate and 3/5 volume of isopropanol, remained at -20°C for 1.5 h and then centrifuged at 3,000 rpm for 20 min at 4°C. The pellet thus obtained was suspended in 2 ml of TE solution (pH 8.0) and incubated at 37°C for 30 min after adding 50 μg of proteinase K and SDS to its final concentration of 0.5%. Resulting transparent solution was extracted with a phenol/chloroform system and precipitated with 2 volume of ethanol. The filamentous precipitate thus obtained was isolated by centrifuging at 3,000 rpm for 10 min at 4°C. After washing with 70% ethanol and drying, the pellet was dissolved in 400 μl of TE solution (pH 8.0). The solution was again treated twice with the phenol/chloroform extraction and ethanol precipitation methods and the finally obtained pellet was dissolved in 20 μl of TE solution (pH 8.0). A reaction solution (total volume, 10 μl) was prepared by mixing 1 μl of the DNA solution thus obtained with 1 μl of BSA (2 mg/ml solution), 1 μl of RNase A (1 mg/ml solution), one unit of *Eco*RI and 1 μl of 10 x *Eco*RI buffer solution which consisted of 0.5 M NaCl, 1 M Tris-HCl buffer (pH 7.5), 70 mM MgCl$_2$ and 70 mM β-mercaptoethanol.

After incubating the reaction solution at 37°C for 1 h, the size of digested cDNA fragments was analyzed by means of an agarose gel electrophoresis. A clone having the longest cDNA was found to be λ HFL 10, but the size of the incorporated cDNA was 1.2 k bp which indicated that this clone did not code for the entire region but part of the P-450 HFLa protein.

## Example 8

### Preparation of a probe

A probe was prepared by labeling cDNA fragment in the positive clone, λ HFL 10, with radioactivity by means of nick translation.

Nick translation was carried out using a commercialized kit (a product of Nippon Gene) and [α-$^{32}$P] dCTP (3,000 Ci/m mol) for the radioactive labeling in accordance with the protocol attached to the kit. The probe thus obtained had a specific activity of $10^8$ to $10^9$ cpm/μg.

## Example 9

### Northern blot analysis

For the purpose of obtaining a cDNA coding for the entire length of P-450 HFLa protein, screening of P-450 HFLa mRNA was carried out again using the probe prepared according to Example 8. Fractions of mRNA were prepared in the same way as described in Example 3. A 20 μl portion of a treatment solution consisting of 40 mM MOPS (pH 7.0), 10 mM sodium acetate, 1 mM EDTA, 2.2 M formaldehyde and 50% formamide, in which 1/20 volume of one of the mRNA fractions was contained, was heated at 55°C for 15 min in order to denature the mRNA. After the denaturation treatment, the solution was mixed with 2 μl of a solution consisting of 50% glycerol, 1 mM EDTA, 0.4% Bromophenol Blue and 0.4% xylene cyanol and then applied to a 1% agarose gel electrophoresis under a constant tension of 15 V/cm. The resulting gel was soaked in 0.3 M sodium citrate solution (pH 7.0) containing 0.3 M NaCl for 1 h and then a nitrocellulose filter which had been soaked in the same solution was laid on bottom side of the soaked gel in order to transfer the mRNA to the filter. The filter was air-dried and then heated at 80°C for 2 h to fix the mRNA thus transferred. The nitrocellulose filter was soaked in a 45 mM sodium citrate solution containing 0.45 M NaCl and 0.1% SDS, transferred into a sealing bag and incubated at 42°C for 4 h in a pre-hybridization solution which consisted of 75 mM sodium citrate, 0.75 M NaCl, 50 mM sodium phosphate (pH 6.5), 50% formamide, 0.1% Ficoll (type 400, trade name of a stabilizer produced by Sigma), 0.1% polyvinyl pyrrolidone, 0.1% BSA and 250 μg/ml of denatured salmon sperm DNA. The incubated pre-hybridization solution in the sealing bag was further mixed with the DNA probe, which had been prepared according to Example 8 and made into single-stranded DNA by thermal denaturation, to its final concentration of 3,000,000 cpm/ml. The mixture was incubated overnight at 42°C to complete hybridization. The filter was removed from the sealing bag, transferred in a 30 mM sodium citrate solution containing 0.3 M NaCl and 0.1% SDS and incubated at 55°C for 30 min. The washing step was repeated twice in order to remove nonspecific adsorbed DNA probe. The filters thus prepared were air-dried and applied to an autoradiography in order to determine a fraction containing the largest amount of P-450 HFLa mRNA.

## Example 10

14

Screening of cDNA library using the probe.

New cDNA library was prepared in the same manner as described in Example 5, using the P-450 HFLa mRNA fraction determined in Example 9. After transfecting *E. coli* Y 1090 with the recombinant phage thus obtained, the transfected cells were mixed with a small volume of the same soft agar medium used in Example 6 and spread on a plate agar medium. A total of 1.5 x 10$^5$ plaques were obtained after incubating the overlay plate overnight at 37°C. Phages in the plaques on the surface of the overlay agar were replicated on a nitrocellulose filter and the filter was air-dried. The dried filter was firstly soaked for 1 min in 0.5 M NaOH solution containing 1.5 M NaCl, neutralized by soaking again in 0.5 M Tris-HCl buffer (pH 8.0) containing 1.5 M NaCl for 5 min and then washed with 2 x SSPE solution (standard 1 x SSPE consists of 0.18 M NaCl, 10 mM NaH$_2$PO$_4$ (pH 7.4) and 1 mM EDTA). The washed filter was air-dried and then heated at 80°C for 2 h in an oven in order to fix the DNA on the filter.

Pre-hybridization was carried out at 60°C for 4 h in 5 x SSC solution (standard 1 x SSC consists of 0.15 M NaCl and 15 mM sodium citrate) containing 0.1% BSA, 0.1% Ficoll 400, 0.1% polyvinyl pyrrolidone, 0.1% SDS and 100 μg/ml of thermally denatured salmon sperm DNA. Hybridization was carried out at 60°C overnight in the 5 x SSC solution which had been mixed with about 1 x 10$^6$ cpm/ml of the DNA probe obtained in Example 8 and 100 μg/ml of the denatured salmon sperm DNA. The filter was then washed by incubating it in 2 x SSC containing 0.1% SDS at 60°C for 30 min. The washing step was repeated twice and then the filter was dried. By applying the filter to an autoradiography, 20 positive clones were found. Each of these clones was purified to a homogeneous level in accordance with the method described in Example 6.- (3). The purified clones were treated in the same manner as described in Example 7 and the size of cDNA incorporated into the purified clones was analyzed electrophoretically. As the result, it was found that a clone, named λHFL 33, contained about 2 k bp of the cDNA which was digested into three *Eco*RI fragments.

A fragment ($P_{vu}$ II - $K_{pn}$ I) with about 4.2 k bp containing entire length of the P-450 HFLa cDNA (ca. 2 k bp) was isolated from the phage DNA of the λHFL 33 clone. A plasmid, named pUC 18 λHFL 33, was obtained by sub-cloning the fragment into the $S_{ma}$ I - $K_{pn}$ I site of a plasmid pUC 18. A recombinant strain, named *E. coli* JM 101 (pUC 18 λHFL 33), was obtained by transforming *E. coli* strain JM 101 with the plasmid pUC 18 λHFL 33. One of the present inventors has deposited this recombinant strain at Fermentation Research Institute, Agency of Industrial Science and Technology, as "FERM BP-2289, Feb., 15, 1989".

Example 11

Subcloning of P-450 HFLa cDNA.

Each of the three *Eco* RI fragments of the λHFL 33 cDNA which had been obtained in Example 10 was transferred from the fragment-containing gel on a DEAE ion exchange membrane filter. The fragment was eluted from the filter with a TE solution containing 1 M NaCl and then recovered from the eluted fractions by phenol/chloroform extraction and ethanol precipitation. The precipitate of each cDNA fragment thus recovered was dissolved in 5 μl of 10 mM TE solution and ligated with plasmid pUC 18 which bad been digested with *Eco*RI and treated with an alkaline phosphatase. *E. coli* JM 101 was transformed with the recombinant plasmid thus obtained and plated on an LB agar medium (LB medium containing 1.5% agar) which had been supplemented with 35 μg/ml of X-gal as the chromogenic indicator. After incubating the plated cells at 37°C overnight, a white colony grown on the plate medium was inoculated into 1.5 ml of the LB medium and cultured at 37°C for 5 h on a shaker. The cultured broth was transferred in an Eppendorf tube and centrifuged at 10,000 x g for 5 min. The resulting pellet was suspended in 100 μl of 25 mM TE solution (pH 8.0, 10 mM EDTA) which had been supplemented with 50 mM glucose and 5 mg/ml of lysozyme. After keeping for 5 min at room temperature, the suspension was mixed gently with 200 μl of a solution of 0.2 N NaOH and 1% SDS and the mixture was then cooled in an ice bath for 5 min. The resulting solution was further mixed with 150 $\mu$l of 3 M potassium acetate solution (pH 5.2), cooled in an ice bath for 5 min and then centrifuged at 10,000 x g for 5 min at 4° C. The supernatant fluid was applied to the phenol/chloroform extraction and ethanol precipitation steps. The plasmid DNA thus obtained was dissolved in TE solution (pH 8.0), treated with 25 $\mu$g/ml (final concentration) of RNase A and again applied to the phenol/chloroform extraction and ethanol precipitation steps. In this way, about 30 $\mu$g of purified plasmid DNA containing each of the three EcoRI fragments was obtained .


Example 12


Construction of restriction enzyme map and determination of nucleotide sequence.


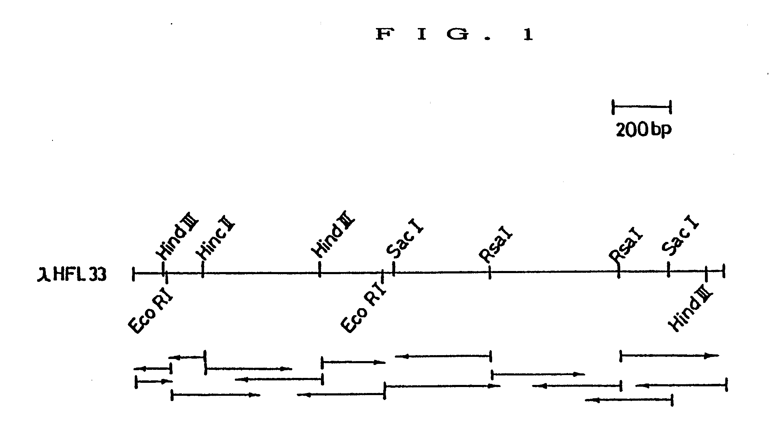

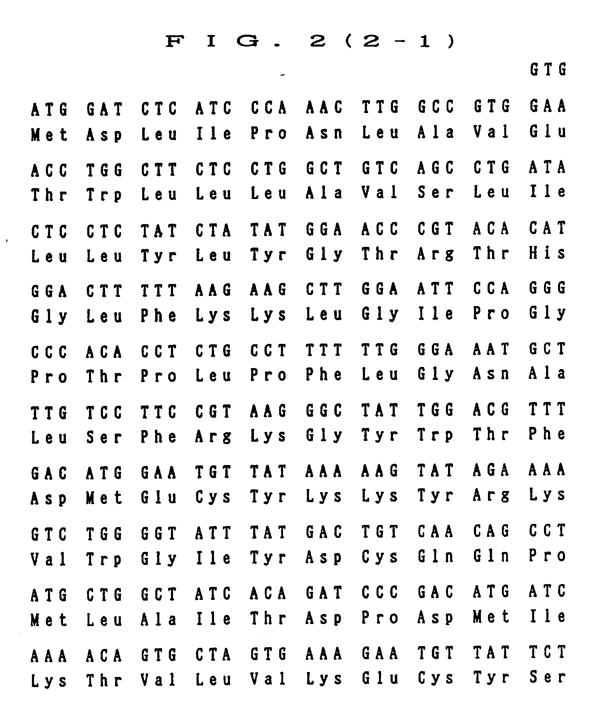

The purified plasmid DNAs obtained in Example 11 by subcloning each of the three EcoRI fragments of λHFL 33 into pUC 18 were named pHFL 33ss, pHFL 33s and pHFL 331 according to the size of the inserts (small, middle and large, respectively). A restriction enzyme map was constructed using cDNA inserts of these three plasmid DNAs and λHFL 33. A 2 $\mu$g portion of the plasmid DNA was cut with 1 $\mu$l of various restriction enzymes (5 - 15 units), the digests were applied to 0.9% agarose gel electrophoresis and a restriction enzyme map was constructed by determining the splitting site of the cDNA for each restriction enzyme on the basis of the number of bands and mobility of each band. A restriction enzyme map of the cDNA insert of λHFL 33 is shown in Fig. 1

Next, the DNA was cut with the restriction enzymes according to the restriction enzyme map and the digests were subcloned into M 13 mp 18 and M 13 mp 19. The subcloned fragments and direction of the sequencing are shown in Fig. 1. Sequencing of the DNA was carried out as follows in accordance with the method of Sanger et al.(J. Mol. Biol., vol. 143, p. 161, 1980). That is, E. coli JM 101 was transformed with a recombinant plasmid of M 13 mp 18 or M 13 mp 19. The transformed cells were mixed with 2.5 ml of a soft agar medium (10 g/l of Bactotryptone, 8 g/l of NaCl and 8 g/l of agar), 40 $\mu$l of 100 mM IPTG and 40 $\mu$l of 2% X-gal, and the mixture was poured on H agar medium (10 g/l of Bactotryptone, 8 g/l of NaCl and 17 g/l of agar) in order to obtain phage plaques. An opaque plaque formed on the medium after culturing was inoculated in 1.5 ml of 2 x TY medium wherein 1/100 volume of an overnight culture broth of E. coli JM 101 had been supplemented in order to propagate the phage. After 5 h culturing of the inoculated medium, cells in the medium were removed by centrifugation and the propagated phages were precipitated by adding 2.5 M NaCl-containing 20% polyethylene glycol to the supernatant fluid and keeping the mixed fluid at room temperature for 15 min. After removing polyethylene glycol by centrifugation, the resulting pellet was dissolved in 100 $\mu$l of TE solution, treated with phenol and centrifuged. To the water layer of the centrifuged sample was added 3 M sodium acetate and ethanol in order to precipitate single-stranded DNA. Resulting pellet was vacuum-dried and the precipitate was suspended in 30 $\mu$l of TE solution and stored in an ice bath.

About 5 $\mu$g of the single-stranded DNA thus obtained (total volume, 7 $\mu$l) was mixed with 1 $\mu$l of M 13 primer (ca. 1.5 ng), 1.5 $\mu$l of 10 x Klenow buffer and 2.5 $\mu$l of 100 mM Tris-HCl buffer (pH 8.0) containing 50 mM MgCl$_2$, in order to complete annealing reaction and formation of complex of template and primer. After annealing, the reaction solution was mixed with 1 $\mu$l of Klenow fragment (1 unit) and 1.5 $\mu$l of [$^{35}$S] dATP αS (10 Ci/ml; relative radioactivity, >600 Ci/m mol). A 2.5 $\mu$l portion of the mixture was transferred in each of four tubes. To each tube was then added 2 $\mu$l of a solution mixture consisting of 80 $\mu$M dCTP, 80 $\mu$M dGTP, 80 $\mu$M dTTP and 8 $\mu$M ddNTP ("N" corresponds to A, G, C or T). The tubes were incubated at 37° C for 15 min. The chase reaction was continued further at 37° C for 15 min after adding 2 $\mu$l of 0.5 mM dNTP solution. The reaction was stopped by adding 4 $\mu$l of a stopper solution which consisted of 95% deionized formamide, 25 mM EDTA (pH 8.0), 0.1% Bromophenol Blue and 0.1% xylene cyanol.

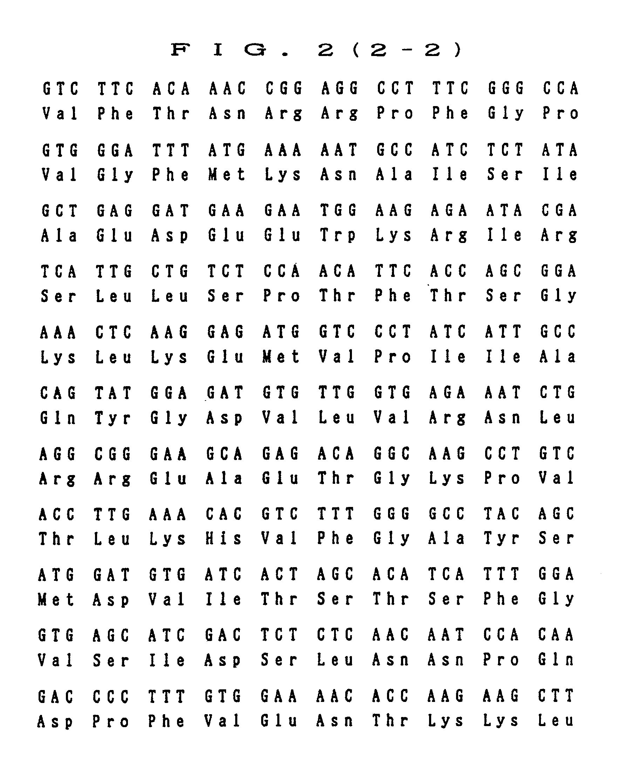

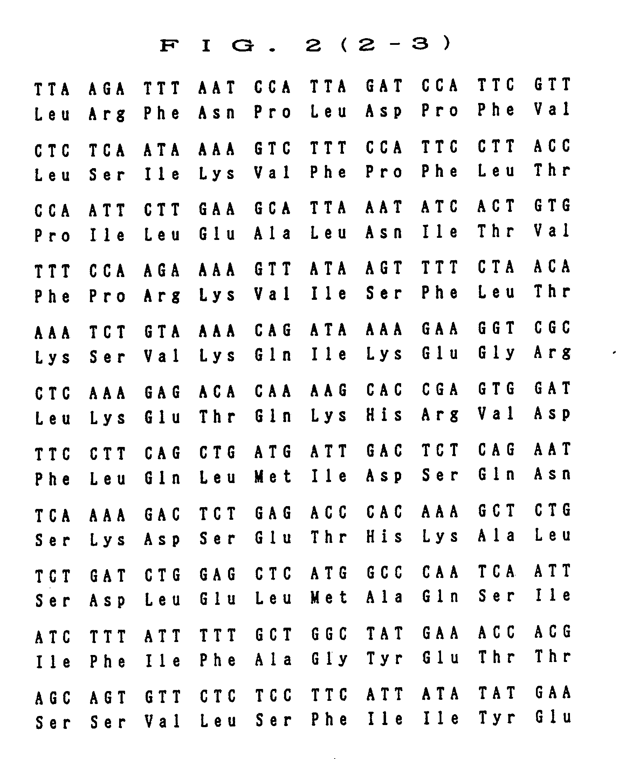

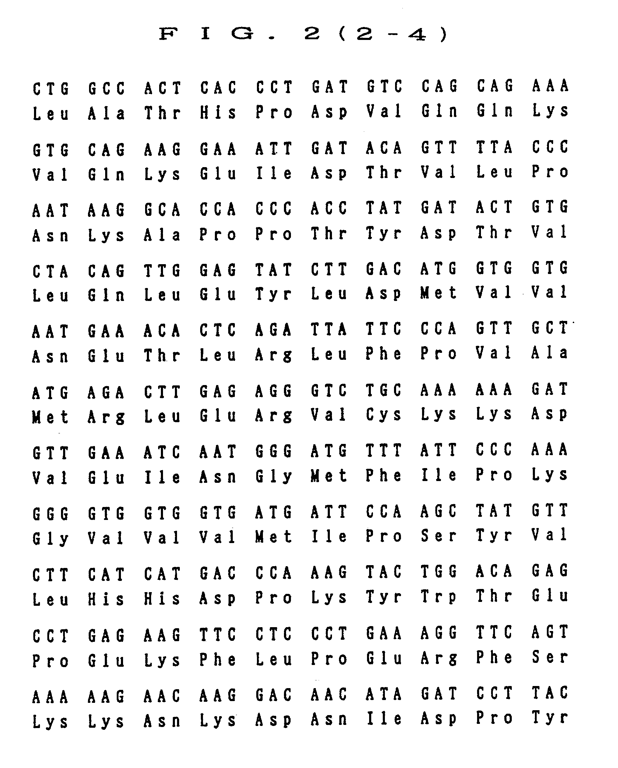

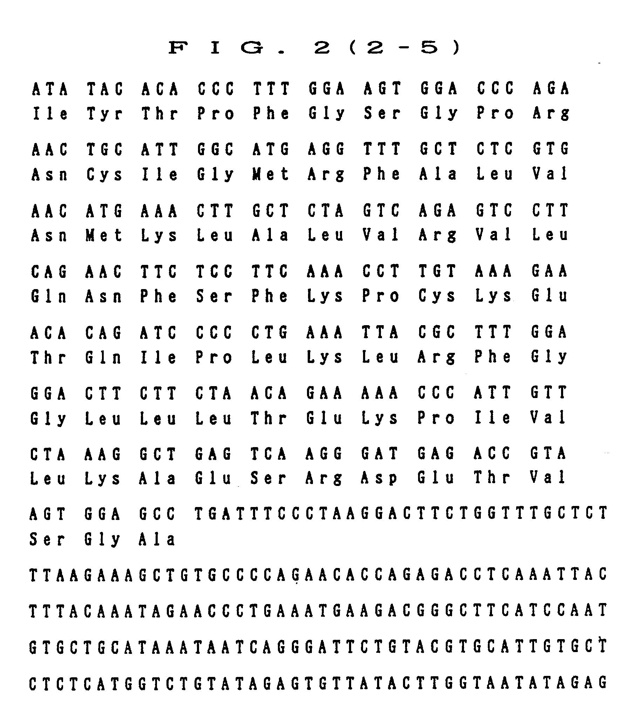

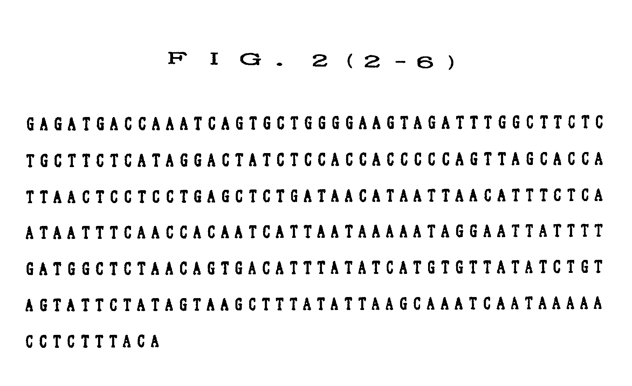

Nucleotide sequence of the DNA was determined by means of electrophoresis and subsequent autoradiography. Result of the sequencing and amino acid sequence deduced from the nucleotide sequence are shown in Fig. 2. It was found that the cDNA insert of the λHFL 33 clone consisted of 1,971 base pairs, the region cording for P-450 HFLa being 1,509 base pairs which correspond to 503 amino acid residues.

As have been described in detail in the foregoing, the present inventors have determined the DNA sequence of a gene coding for the P-450 HFLa protein specific to human fetal livers and, on the basis of the result, deduced the amino acid sequence of said protein. Said gene is applicable to the DNA diagnosis of such diseases as gynecological malignant tumors. A large scale production of a protein having the amino acid sequence of the present invention may be accomplished by means of the recombinant DNA technology using said gene. Moreover, an antibody which is specific to the P-450 HFLa may be prepared using all or at least a portion of the amino acid sequence of the present invention and applied to the diagnosis.

**Claims**

1. Cytochrome P-450 HFLa protein essentially free of other proteins of human origin.

2. Cytochrome P-450 HFLa protein according to claims 1 wherein said protein is produced by a recombinant host cell.

3. Cytochrome P-450 HFLa protein comprising at least a portion of an amino acid sequence represented by sequence [I]:

Met Asp Leu Ile Pro Asn Leu Ala Val Glu
Thr Trp Leu Leu Leu Ala Val Ser Leu Ile
Leu Leu Tyr Leu Tyr Gly Thr Arg Thr His
Gly Leu Phe Lys Lys Leu uly Ile Pro Gly
Pro Thr Pro Leu Pro Phe Leu Gly Asn Ala
Leu Ser Phe Arg Lys Gly Tyr Trp Thr Phe
Asp Met Glu Cys Tyr Lys Lys Tyr Arg Lys
Val Trp Gly Ile Tyr Asp Cys Gln Gln Pro
Met Leu Ala Ile Thr Asp Pro Asp Met Ile
Lys Thr Val Leu Val Lys Glu Cys Tyr Ser
Val Phe Thr Asn Arg Arg Pro Phe Gly Pro
Val Gly Phe Met Lys Asn Ala Ile Ser Ile
Ala Glu Asp Glu Glu Trp Lys Arg Ile Arg
Ser Leu Leu Ser Pro Thr Phe Thr Ser Gly
Lys Leu Lys Glu Met Val Pro Ile Ile Ala
Gln Tyr Gly Asp Val Leu Val Arg Asn Leu
Arg Arg Glu Ala Glu Thr Gly Lys Pro Val
Thr Leu Lys His Val Phe Gly Ala Tyr Ser
Met Asp Val Ile Thr Ser Thr Ser Phe Gly
Val Ser Ile Asp Ser Leu Asn Asn Pro Gln
Asp Pro Phe Val Glu Asn Thr Lys Lys Leu
Leu Arg Phe Asn Pro Leu Asp Pro Phe Val
Leu Ser Ile Lys Val Phe Pro Phe Leu Thr
Pro Ile Leu Glu Ala Leu Asn Ile Thr Val
Phe Pro Arg Lys Val Ile Ser Phe Leu Thr
Lys Ser Val Lys Gln Ile Lys Glu Gly Arg
Leu Lys Glu Thr Gln Lys His Arg Val Asp
Phe Leu Gln Leu Met Ile Asp Ser Gln Asn
Ser Lys Asp Ser Glu Thr His Lys Ala Leu
Ser Asp Leu Glu Leu Met Ala Gln Ser Ile
Ile Phe Ile Phe Ala Gly Tyr Glu Thr Thr
Ser Ser Val Leu Ser Phe Ile Ile Tyr Glu
Leu Ala Thr His Pro Asp Val Gln Gln Lys
Val Gln Lys Glu Ile Asp Thr Val Leu Pro
Asn Lys Ala Pro Pro Thr Tyr Asp Thr Val
Leu Gln Leu Glu Tyr Leu Asp Met Val Val
Asn Glu Thr Leu Arg Leu Phe Pro Val Ala
Met Arg Leu Glu Arg Val Cys Lys Lys Asp
Val Glu Ile Asn Gly Met Phe Ile Pro Lys
Gly Val Val Val Met Ile Pro Ser Tyr Val
Leu His His Asp Pro Lys Tyr Trp Thr Glu
Pro Glu Lys Phe Leu Pro Glu Arg Phe Ser
Lys Lys Asn Lys Asp Asn Ile Asp Pro Tyr
Ile Tyr Thr Pro Phe Gly Ser Gly Pro Arg

Asn Cys Ile Gly Met Arg Phe Ala Leu Val
Asn Met Lys Leu Ala Leu Val Arg Val Leu
Gln Asn Phe Ser Phe Lys Pro Cys Lys Glu
Thr Gln Ile Pro Leu Lys Leu Arg Phe Gly
Gly Leu Leu Leu Thr Glu Lys Pro Ile Val
Leu Lys Ala Glu Ser Arg Asp Glu Thr Val
Ser Gly Ala      [ I ]

4. A DNA sequence encoding the cytochrome P-450 HFLa protein according to any of the claims 1 to 3.

5. A DNA sequence comprising at least a portion of a DNA sequence represented by sequence [II]:

ATG GAT CTC ATC CCA AAC TTG GCC GTG GAA
ACC TGG CTT CTC CTG GCT GTC AGC CTG ATA
CTC CTC TAT CTA TAT GGA ACC CGT ACA CAT
GGA CTT TTT AAG AAG CTT GGA ATT CCA GGG
CCC ACA CCT CTG CCT TTT TTG GGA AAT GCT
TTG TCC TTC cGT AAG GGC TAT TGG ACG TTT
GAC ATG GAA TGT TAT AAA AAG TAT AGA AAA
GTC TGG GGT ATT TAT GAC TGT CAA CAG CCT
ATG CTG GCT ATC ACA GAT CCC GAC ATG ATC
AAA ACA GTG CTA GTG AAA GAA TGT TAT TCT
GTC TTC ACA AAC CGG AGG CCT TTC GGG CCA
GTG GGA TTT ATG AAA AAT GCC ATC TCT ATA
GCT GAG GAT GAA GAA TGG AAG AGA ATA CGA
TCA TTG CTG TCT CCA ACA TTC ACC AGC GGA
AAA CTC AAG GAG ATG GTC CCT ATC ATT GCC
CAG TAT GGA GAT GTG TTG GTG AGA AAT CTG
AGG CGG GAA GCA GAG ACA GGC AAG CCT GTC
ACC TTG AAA CAC GTC TTT GGG GCC TAC AGC
ATG GAT GTG ATC ACT AGC ACA TCA TTT GGA
GTG AGC ATC GAC TCT CTC AAC AAT CCA CAA
GAC CCC TTT GTG GAA AAC ACC AAG AAG CTT
TTA AGA TTT AAT CCA TTA GAT CCA TTC GTT
CTC TCA ATA AAA GTC TTT CCA TTC CTT ACC
CCA ATT CTT GAA GCA TTA AAT ATC ACT GTG
TTT CCA AGA AAA GTT ATA AGT TTT CTA ACA
AAA TCT GTA AAA CAG ATA AAA GAA GGT CGC
CTC AAA GAG ACA CAA AAG CAC CGA GTG GAT
TTC CTT CAG CTG ATG ATT GAC TCT CAG AAT
TCA AAA GAC TCT GAG ACC CAC AAA GCT CTG
TCT GAT CTG GAG CTC ATG GCC CAA TCA ATT
ATC TTT ATT TTT GCT GGC TAT GAA ACC ACG
AGC AGT GTT CTC TCC TTC ATT ATA TAT GAA
CTG GCC ACT CAC CCT GAT GTC CAG CAG AAA
GTG CAG AAG GAA ATT GAT ACA GTT TTA CCC
AAT AAG GCA CCA CCC ACC TAT GAT ACT GTG
CTA CAG TTG GAG TAT CTT GAC ATG GTG GTG
AAT GAA ACA CTC AGA TTA TTC CCA GTT GCT
ATG AGA CTT GAG AGG GTC TGC AAA AAA GAT
GTT GAA ATC AAT GGG ATG TTT ATT CCC AAA
GGG GTG GTG GTG ATG ATT CCA AGC TAT GTT
CTT CAT CAT GAC CCA AAG TAC TGG ACA GAG
CCT GAG AAG TTC CTC CCT GAA AGG TTC AGT
AAA AAG AAC AAG GAC AAC ATA GAT CCT TAC
ATA TAC ACA CCC TTT GGA AGT GGA CCC AGA
AAC TGC ATT GGC ATG AGG TTT GCT CTC GTG
AAC ATG AAA CTT GCT CTA GTC AGA GTC CTT
CAG AAC TTC TCC TTC AAA CCT TGT AAA GLA
ACA CAG ATC CCC CTG AAA TTA CGC TTT GGA

GGA CTT CTT CTA ACA GAA AAA CCC ATT GTT
CTA AAG GCT GAG OCA AGG GAT GAG ACC GTA
AGT GGA GCC TGA       [ II ]
optionally having at least one base substituted by degeneracy of genetic codon.

6. A DNA sequence complementary to said DNA sequence of claim 4 or 5.

7. A process for producing cytochrome P-450 HFLa protein wherein a DNA sequence encoding the cytochrome P-450 HFLa protein is expressed in a recombinant host cell.

FIG. 1

# F I G . 2 ( 2 - 1 )

```
                                                    GTG

ATG GAT CTC ATC CCA AAC TTG GCC GTG GAA
Met Asp Leu Ile Pro Asn Leu Ala Val Glu

ACC TGG CTT CTC CTG GCT GTC AGC CTG ATA
Thr Trp Leu Leu Leu Ala Val Ser Leu Ile

CTC CTC TAT CTA TAT GGA ACC CGT ACA CAT
Leu Leu Tyr Leu Tyr Gly Thr Arg Thr His

GGA CTT TTT AAG AAG CTT GGA ATT CCA GGG
Gly Leu Phe Lys Lys Leu Gly Ile Pro Gly

CCC ACA CCT CTG CCT TTT TTG GGA AAT GCT
Pro Thr Pro Leu Pro Phe Leu Gly Asn Ala

TTG TCC TTC CGT AAG GGC TAT TGG ACG TTT
Leu Ser Phe Arg Lys Gly Tyr Trp Thr Phe

GAC ATG GAA TGT TAT AAA AAG TAT AGA AAA
Asp Met Glu Cys Tyr Lys Lys Tyr Arg Lys

GTC TGG GGT ATT TAT GAC TGT CAA CAG CCT
Val Trp Gly Ile Tyr Asp Cys Gln Gln Pro

ATG CTG GCT ATC ACA GAT CCC GAC ATG ATC
Met Leu Ala Ile Thr Asp Pro Asp Met Ile

AAA ACA GTG CTA GTG AAA GAA TGT TAT TCT
Lys Thr Val Leu Val Lys Glu Cys Tyr Ser
```

F I G .   2 ( 2 - 2 )

```
GTC   TTC   ACA   AAC   CGG   AGG   CCT   TTC   GGG   CCA
Val   Phe   Thr   Asn   Arg   Arg   Pro   Phe   Gly   Pro

GTG   GGA   TTT   ATG   AAA   AAT   GCC   ATC   TCT   ATA
Val   Gly   Phe   Met   Lys   Asn   Ala   Ile   Ser   Ile

GCT   GAG   GAT   GAA   GAA   TGG   AAG   AGA   ATA   CGA
Ala   Glu   Asp   Glu   Glu   Trp   Lys   Arg   Ile   Arg

TCA   TTG   CTG   TCT   CCA   ACA   TTC   ACC   AGC   GGA
Ser   Leu   Leu   Ser   Pro   Thr   Phe   Thr   Ser   Gly

AAA   CTC   AAG   GAG   ATG   GTC   CCT   ATC   ATT   GCC
Lys   Leu   Lys   Glu   Met   Val   Pro   Ile   Ile   Ala

CAG   TAT   GGA   GAT   GTG   TTG   GTG   AGA   AAT   CTG
Gln   Tyr   Gly   Asp   Val   Leu   Val   Arg   Asn   Leu

AGG   CGG   GAA   GCA   GAG   ACA   GGC   AAG   CCT   GTC
Arg   Arg   Glu   Ala   Glu   Thr   Gly   Lys   Pro   Val

ACC   TTG   AAA   CAC   GTC   TTT   GGG   GCC   TAC   AGC
Thr   Leu   Lys   His   Val   Phe   Gly   Ala   Tyr   Ser

ATG   GAT   GTG   ATC   ACT   AGC   ACA   TCA   TTT   GGA
Met   Asp   Val   Ile   Thr   Ser   Thr   Ser   Phe   Gly

GTG   AGC   ATC   GAC   TCT   CTC   AAC   AAT   CCA   CAA
Val   Ser   Ile   Asp   Ser   Leu   Asn   Asn   Pro   Gln

GAC   CCC   TTT   GTG   GAA   AAC   ACC   AAG   AAG   CTT
Asp   Pro   Phe   Val   Glu   Asn   Thr   Lys   Lys   Leu
```

# F I G . 2 ( 2 - 3 )

```
TTA   AGA   TTT   AAT   CCA   TTA   GAT   CCA   TTC   GTT
Leu   Arg   Phe   Asn   Pro   Leu   Asp   Pro   Phe   Val

CTC   TCA   ATA   AAA   GTC   TTT   CCA   TTC   CTT   ACC
Leu   Ser   Ile   Lys   Val   Phe   Pro   Phe   Leu   Thr

CCA   ATT   CTT   GAA   GCA   TTA   AAT   ATC   ACT   GTG
Pro   Ile   Leu   Glu   Ala   Leu   Asn   Ile   Thr   Val

TTT   CCA   AGA   AAA   GTT   ATA   AGT   TTT   CTA   ACA
Phe   Pro   Arg   Lys   Val   Ile   Ser   Phe   Leu   Thr

AAA   TCT   GTA   AAA   CAG   ATA   AAA   GAA   GGT   CGC
Lys   Ser   Val   Lys   Gln   Ile   Lys   Glu   Gly   Arg

CTC   AAA   GAG   ACA   CAA   AAG   CAC   CGA   GTG   GAT
Leu   Lys   Glu   Thr   Gln   Lys   His   Arg   Val   Asp

TTC   CTT   CAG   CTG   ATG   ATT   GAC   TCT   CAG   AAT
Phe   Leu   Gln   Leu   Met   Ile   Asp   Ser   Gln   Asn

TCA   AAA   GAC   TCT   GAG   ACC   CAC   AAA   GCT   CTG
Ser   Lys   Asp   Ser   Glu   Thr   His   Lys   Ala   Leu

TCT   GAT   CTG   GAG   CTC   ATG   GCC   CAA   TCA   ATT
Ser   Asp   Leu   Glu   Leu   Met   Ala   Gln   Ser   Ile

ATC   TTT   ATT   TTT   GCT   GGC   TAT   GAA   ACC   ACG
Ile   Phe   Ile   Phe   Ala   Gly   Tyr   Glu   Thr   Thr

AGC   AGT   GTT   CTC   TCC   TTC   ATT   ATA   TAT   GAA
Ser   Ser   Val   Leu   Ser   Phe   Ile   Ile   Tyr   Glu
```

# F I G . 2 ( 2 - 4 )

```
CTG   GCC   ACT   CAC   CCT   GAT   GTC   CAG   CAG   AAA
Leu   Ala   Thr   His   Pro   Asp   Val   Gln   Gln   Lys

GTG   CAG   AAG   GAA   ATT   GAT   ACA   GTT   TTA   CCC
Val   Gln   Lys   Glu   Ile   Asp   Thr   Val   Leu   Pro

AAT   AAG   GCA   CCA   CCC   ACC   TAT   GAT   ACT   GTG
Asn   Lys   Ala   Pro   Pro   Thr   Tyr   Asp   Thr   Val

CTA   CAG   TTG   GAG   TAT   CTT   GAC   ATG   GTG   GTG
Leu   Gln   Leu   Glu   Tyr   Leu   Asp   Met   Val   Val

AAT   GAA   ACA   CTC   AGA   TTA   TTC   CCA   GTT   GCT
Asn   Glu   Thr   Leu   Arg   Leu   Phe   Pro   Val   Ala

ATG   AGA   CTT   GAG   AGG   GTC   TGC   AAA   AAA   GAT
Met   Arg   Leu   Glu   Arg   Val   Cys   Lys   Lys   Asp

GTT   GAA   ATC   AAT   GGG   ATG   TTT   ATT   CCC   AAA
Val   Glu   Ile   Asn   Gly   Met   Phe   Ile   Pro   Lys

GGG   GTG   GTG   GTG   ATG   ATT   CCA   AGC   TAT   GTT
Gly   Val   Val   Val   Met   Ile   Pro   Ser   Tyr   Val

CTT   CAT   CAT   GAC   CCA   AAG   TAC   TGG   ACA   GAG
Leu   His   His   Asp   Pro   Lys   Tyr   Trp   Thr   Glu

CCT   GAG   AAG   TTC   CTC   CCT   GAA   AGG   TTC   AGT
Pro   Glu   Lys   Phe   Leu   Pro   Glu   Arg   Phe   Ser

AAA   AAG   AAC   AAG   GAC   AAC   ATA   GAT   CCT   TAC
Lys   Lys   Asn   Lys   Asp   Asn   Ile   Asp   Pro   Tyr
```

# F I G . 2 ( 2 - 5 )

```
ATA  TAC  ACA  CCC  TTT  GGA  AGT  GGA  CCC  AGA
Ile  Tyr  Thr  Pro  Phe  Gly  Ser  Gly  Pro  Arg

AAC  TGC  ATT  GGC  ATG  AGG  TTT  GCT  CTC  GTG
Asn  Cys  Ile  Gly  Met  Arg  Phe  Ala  Leu  Val

AAC  ATG  AAA  CTT  GCT  CTA  GTC  AGA  GTC  CTT
Asn  Met  Lys  Leu  Ala  Leu  Val  Arg  Val  Leu

CAG  AAC  TTC  TCC  TTC  AAA  CCT  TGT  AAA  GAA
Gln  Asn  Phe  Ser  Phe  Lys  Pro  Cys  Lys  Glu

ACA  CAG  ATC  CCC  CTG  AAA  TTA  CGC  TTT  GGA
Thr  Gln  Ile  Pro  Leu  Lys  Leu  Arg  Phe  Gly

GGA  CTT  CTT  CTA  ACA  GAA  AAA  CCC  ATT  GTT
Gly  Leu  Leu  Leu  Thr  Glu  Lys  Pro  Ile  Val

CTA  AAG  GCT  GAG  TCA  AGG  GAT  GAG  ACC  GTA
Leu  Lys  Ala  Glu  Ser  Arg  Asp  Glu  Thr  Val

AGT  GGA  GCC  TGATTTCCCTAAGGACTTCTGGTTTGCTCT
Ser  Gly  Ala

TTAAGAAAGCTGTGCCCCAGAACACCAGAGACCTCAAATTAC

TTTACAAATAGAACCCTGAAATGAAGACGGGCTTCATCCAAT

GTGCTGCATAAATAATCAGGGATTCTGTACGTGCATTGTGCT

CTCTCATGGTCTGTATAGAGTGTTATACTTGGTAATATAGAG
```

# F I G . 2 ( 2 - 6 )

GAGATGACCAAATCAGTGCTGGGGAAGTAGATTTGGCTTCTC

TGCTTCTCATAGGACTATCTCCACCACCCCCAGTTAGCACCA

TTAACTCCTCCTGAGCTCTGATAACATAATTAACATTTCTCA

ATAATTTCAACCACAATCATTAATAAAAATAGGAATTATTTT

GATGGCTCTAACAGTGACATTTATATCATGTGTTATATCTGT

AGTATTCTATAGTAAGCTTTATATTAAGCAAATCAATAAAAA

CCTCTTTACA

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | THE JOURNAL OF BIOLOGICAL CHEMISTRY, vol. 262, no. 28, 5th October 1987, pages 13534-13537, The American Society for Biochemistry and Molecular Biology, Inc., Bethesda, MD, US; M. KITADA et al.: "P-450 HFLa, a form of cytochrome P-450 purified from human fetal livers, is the 16alpha-hydroxylase of dehydroepiandrosterone 3-sulfate" * Whole document * | 1 | C 12 N 15/53<br>C 12 N 9/02 |
| Y | IDEM | 2-7 | |
| Y | DNA, vol. 7, no. 2, 1988, pages 79-86, Mary Ann Liebert, Inc.; F.J. GONZALEZ et al.: "Human P450PCN1: sequence, chromosome localization, and direct evidence through cDNA expression that P450PCN1 is nifedipine oxidase" * Whole document * | 2-7 | |
| X,D | ARCHIVES OF BIOCHEMISTRY AND BIOPHYSICS, vol. 241, no. 1, 15th August 1985, pages 275-280, Academic Press, Inc., New York, US; M. KITADA et al.: "Purification and properties of cytochrome P-450 from homogenates of human fetal livers" * Whole document * | 1 | TECHNICAL FIELDS SEARCHED (Int. Cl.5)<br><br>C 12 N |
| X,P | JOURNAL OF BIOCHEMISTRY, vol. 105, February 1989, pages 161-163, Tokyo, JP; M. KOMORI et al.: "Molecular cloning and sequence analysis of cDNA containing the entire coding region for human fetal liver cytochrome P-450" * Whole document * | 1-7 | |

The present search report has been drawn up for all claims

| Place of search | Date of completion of the search | Examiner |
|---|---|---|
| THE HAGUE | 26-04-1990 | LE CORNEC N.D.R. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................

& : member of the same patent family, corresponding document

EPO FORM 1503 03.82 (P0401)